Europäisches Patentamt

(19) European Patent Office

Office européen des brevets

(11) **EP 0 837 091 A1**

(12) **EUROPEAN PATENT APPLICATION**
published in accordance with Art. 158(3) EPC

(43) Date of publication:
22.04.1998 Bulletin 1998/17

(51) Int. Cl.[6]: **C08J 9/28**, A61L 31/00

(86) International application number:
PCT/JP97/01447

(21) Application number: 97919691.2

(22) Date of filing: 24.04.1997

(87) International publication number:
WO 97/41169 (06.11.1997 Gazette 1997/47)

(84) Designated Contracting States:
DE FR GB

(30) Priority: 26.04.1996 JP 130722/96

(71) Applicants:
• Toho Rayon Co., Ltd.
Chuo-ku, Tokyo 103 (JP)
• Hogy Medical Co., Ltd.
Tokyo 113 (JP)

(72) Inventors:
• YAMAGUCHI, Shuichiro,
Toho Rayon Co., Ltd.
Itano-gun, Tokushima 771-02 (JP)

• MOTOKI, Tetsuya,
Toho Rayon Co., Ltd.
Itano-gun, Tokushima 771-02 (JP)
• MURAMATSU, Teruaki,
Toho Rayon Co., Ltd.
Itano-gun, Tokushima 771-02 (JP)
• ISHIKAWA, Nobuhiro
Kashiwa-shi, Chiba 277 (JP)

(74) Representative: Fiener, Josef
Patentanwälte
Kahler, Käck, Fiener et col.,
P.O. Box 12 49
87712 Mindelheim (DE)

(54) **CELLULOSE SPONGE AND METHOD OF MANUFACTURING SAME**

(57) Cellulose sponge, which has a high alcohol aqueous solution absorption, exhibits suitable flexibility irrespective of whether a chemical is contained, suppresses generation of lint and is suitable for medical care, and a method of manufacturing the same. The method of manufacturing cellulose sponge comprises heating, solidifying and forming a sponge undiluted solution, which contains viscose and crystal mirabilite but does not contain reinforcing fibre, in a die (forming process), and performing aftertreatment to manufacture cellulose sponge, the forming process comprising at least a process of holding the sponge undiluted solution at at least 35 °C but at most 70 °C for at least 30 minutes but at most 24 hours. The resulting cellulose sponge has a rate of alcohol aqueous solution absorption of at least 1500 % but at most 3000 %, an apparent density of at least 0.01 g/ml but at most 0.07 g/ml in a dry state, flexibility, and a strength of at least 8 g/mm$^2$ but at most 25 g/mm$^2$ when made wet by water.

EP 0 837 091 A1

## Description

Technical Field

The present invention relates to a cellulose sponge preferable for medical use and a method of producing the same. The present invention relates to a cellulose sponge preferable for medical use and a method of producing the same, characterized in that the cellulose sponge of the present invention has a larger fluid absorption ratio of aqueous alcohol solutions and can keep softness with no hardening even after the sponge is wet in aqueous alcohol solutions; additionally, the sponge can retain its high strength despite no reinforcing fiber contained therein but still has flexibility with no generation of lint during use.

Background Art

Cellulose sponges have been produced generally, by a method comprising mixing a reinforcing fiber and crystal mirabilite with viscose to prepare a raw sponge solution, heating and solidifying the solution, and subsequently effecting post-treatment comprising washing treatment in water and acid treatment, to produce a regenerated- cellulose sponge with the cellulose backbone. By the method of producing the cellulose sponges described above, crystal mirabilite is solubilized through the washing treatment in water to contribute the formation of pores, while the reinforcing fiber contributes to such a strength that the sponge shape can be maintained.

Because cellulose sponges thus produced have poor softness and are so hard after drying, conventionally, a variety of attempts have been made to improve the softness. For example, the particle diameter of crystal mirabilite to be added to viscose is a factor determining the pore size of the cellulose sponge; if crystal mirabilite of a particle diameter as small as possible is used, the cell of the resulting cellulose is smaller and the thickness of the cell wall of the resulting sponge is thinner and more uniform, even if the same amount of crystal mirabilite is used. It has been known that the selection of the particle diameter is one effective means for improving the softness of cellulose sponge (Japanese Patent Laid-open Sho 49-74763).

As another method of imparting softness, a method has been known, comprising adding a specific surfactant together with a polyol compound (for example, Japanese Patent Laid-open No. Hei 02-135235, Japanese Patent Laid-open No. Hei 04-202244).

In recent years, attempts have been made to use cellulose sponge as a medical sponge. For medical use, such sponge is in direct contact to a human affected part, for example for disinfection of cut and abrasion and disinfection of a diseased part prior to surgery, as fluid absorbing materials during surgery, and for disinfection of wounds after surgery and disinfection of mucous membranes of eye and vagina. The sponge is mainly used at states impregnated with aqueous ethanol solutions and aqueous isopropyl alcohol solutions of about 70 %, or povidone.

However, cellulose has an essential property such that once in contact to alcohol, cellulose gets hard. Therefore, even the conventional cellulose sponges have a property such that the sponges are not swollen even if moistened with an aqueous alcohol solution from its dry state; or the cellulose sponges swollen in water get hard when alcohol is added to the sponges. In any of the cases, the presence of alcohol damages the softness and absorption potency of cellulose sponge, disadvantageously for its application to medical use.

Still further, when conventional cellulose sponges are used for medical use, the reinforcing fiber contained therein is dissociated and turns lint during rubbing a diseased part, which then attaches the part. Therefore, the cellulose sponges lack satisfactory properties for medical use. However, cellulose sponges produced from viscose with no addition of any reinforcing fiber so as to avoid such disadvantageous dissociation of the lint occurring due to the presence of the reinforcing fiber, have so lower strength that the sponges are fractured during handling for example for rubbing a diseased part, so these sponges have insufficient properties for medical use.

Additionally, products from these conventional cellulose sponges are of given shapes through sectioning and the like during molding, but the cut ends of the pore wall in the cellulose sponges in a matrix make their appearance in the form of whisker in the cut (the cut face) during sectioning, which causes lint generation. Such tendency is prominent when a molded article is cut from a large molded article to prepare a desirable cellulose sponge product.

Based on these reasons, the conventional cellulose sponges disadvantageously are not commonly used for medical use.

Thus, it is an object of the present invention to provide a cellulose sponge preferable for medical use and a method of producing the same, characterized in that the cellulose sponge has a larger fluid absorption ratio of aqueous alcohol solutions and can keep appropriate softness irrespective of the presence or absence of alcohol as a disinfectant agent and eliminate the generation of lint due to the presence or absence of a reinforcing fiber; in addition to the above object, it is the other object of the present invention to provide a cellulose sponge where the generation of lint due to the cut opening via cutting is suppressed as much as possible.

Disclosure of the Invention

Although cellulose essentially has a property to get hard when in contact to alcohol, the present inventors have found that under specific manufacturing conditions, a cellulose sponge having a higher fluid absorption ratio of aqueous alcohol solutions and capable of retaining softness even after absorption of aqueous alcohol solutions can be produced. Additionally, the present inventors have found that a cellulose sponge with suppressed generation of lint can be produced. The cellulose sponge produced by the manufacturing method of the present invention has unique properties which have never been found in conventional sponges.

More specifically, the cellulose sponge of the present invention substantially comprises a regenerated cellulose with no reinforcing fiber contained therein, characterized in that the sponge has the following properties.

1. Fluid absorption ratio of aqueous alcohol solutions should be 1,500 % or more to 3,000 % or less.
2. Apparent density at the dry state is 0.01 g/ml or more to 0.07 g/ml or less.

In addition to the properties described above, the cellulose sponge of the present invention characteristically has a strength of 8 g/mm$^2$ or more to 25 g/mm$^2$ or less when moistened with water.

In accordance with the present invention, the individual parameters are defined as described below. Fluid absorption ratio of aqueous alcohol solutions: swelling dry cellulose sponge (A in g) in an aqueous 70 vol. % ethanol solution on a watch glass under no load for one minute, draining excess aqueous solution, and weighing the weight (B in g), the ratio of the weight to the cellulose sponge weight is determined by the following formula;

$$\text{Fluid absorption ratio of aqueous alcohol solutions (\%)} = [(B - A)/A] \times 100 \qquad \text{Formula 1}$$

Strength when moistened in water: measuring the break strength of water-impregnated cellulose sponge at a water content of 200 % by weight under a condition of a tensile rate of 200 mm/min by a tensile strength tester manufactured by Instron, Co. Ltd., the value of the measured strength is defined as the strength when moistened in water.

The cellulose sponge of the present invention preferably has cut area as less as possible so as to suppress lint generation during the use; for example, the sponge has a ratio of the area of the cut face to the total surface area being 50 % or less.

Another cellulose sponge of the present invention is preferably of a shape of a columnar body, which is advantageous for industrial mass production; the sponge has cut face approximately in a vertical direction to the axial direction, with an L/D ratio of 0.5 to 2 wherein L and D represent the length along the axial direction and the diameter of the columnar body, respectively, thereby producing preferable products with less cut face for medical use.

Generally, cellulose sponges for medical use such as disinfection are supplied at dry and compact state to consumers, and are to be swollen prior to use. As very important factors of such cellulose sponges, accordingly, the sponges should have a higher fluid absorption ratio of aqueous alcohol solutions and higher strength such that the cellulose sponges never be fractured when the sponges at a state after absorption of alcohol solutions are applied to diseased parts.

Because the cellulose sponge of the present invention has the characteristic properties as described above, the cellulose sponge has a higher fluid absorption ratio of aqueous alcohol solutions and keeps softness together with sufficient strength, even after the sponge is moistened in aqueous alcohol solutions. Thus, the cellulose sponge of the present invention is preferably used for medical use. Such properties have never been observed in the conventional cellulose sponges.

Additionally, the method of producing the cellulose sponge in accordance with the present invention comprises heating and solidifying a raw sponge solution containing viscose and crystal mirabilite with no reinforcing fiber contained therein for molding in a mold (molding process), and post-treating the resulting molded article to produce the cellulose sponge, wherein the molding process is characterized in that the raw sponge solution is subjected to a temperature holding process for holding the solution at 35 °C or more to 70 °C or less for 30 minutes or more to 24 hours or less, thereafter completely solidifying the resulting product. The cellulose sponge recovered by the method characteristically has the individual properties described above.

In accordance with the present invention, more specifically, it has been found that the adjustment of the solidification temperature at the molding process is a significant requirement for producing the cellulose sponge with a higher fluid absorption ratio of aqueous alcohol solutions and softness with no hardening even in aqueous alcohol solutions.

The method of producing the cellulose sponge in accordance with the present invention is characterized in the molding process including a first temperature holding process (low-temperature solidification process) at 35 °C or more to 70 °C or less, preferably 35 °C or more to 45 °C or less for 30 minutes or more to 24 hours or less, which is a desirable condition for producing a cellulose sponge with soft touch, good recovery rate from swelling in water or alcohol, less contraction after heating solidification during the production process, whereby a low-density product with smooth surface can be produced, and soft touch even when ethanol of about 70 % is absorbed into the sponge.

An preferable embodiment of the molding process further comprises a second temperature holding process (high-temperature solidification process) within 90 °C or more to 100 °C or less, following the first temperature holding process (low-temperature solidification process), advantageously for realizing industrially meaningful production rate.

Brief Description of the Drawings

Fig.1 is a flow sheet depicting the general method of producing the cellulose sponge of the present invention;

Fig.2 is one example of the shape of the cellulose sponge of the present invention, which is of a cylindrical shape;

Fig.3 depicts the state of the cellulose sponge in Fig.2, cut along the direction A-A (axial direction);

Fig.4 is a scanning electron microscopic photograph of the surface of the cellulose sponge of the present invention at 70 magnification, representing the surface of the periphery of Fig.2;

Fig.5 is a scanning electron microscopic photograph of the surface of the cellulose sponge of the present invention at 70 magnification, representing the surface of the cross section of Fig.2;

Fig.6 is a scanning electron microscopic photograph of the surface of the cellulose sponge of the present invention at 70 magnification, representing the ridgeline of Fig.2; and

Fig.7 is a scanning electron microscopic photograph of the surface of the cellulose sponge of the present invention at 70 magnification, representing the longitudinal section of Fig.3.

Description of Symbols

1:      viscose
2:      crystal mirabilite
3-1:    surfactant
3-2:    polyol compound
4:      mixing process
5:      molding process
6:      first temperature holding process
7:      second temperature holding process
8:      mold releasing process
9:      post-treatment process
10:     drying process
11:     cutting process
12:     cellulose sponge product
21:     surface of periphery
22:     cross section
23:     ridgeline
24:     longitudinal section
D:      raw sponge solution

Best Mode of Carrying out the Invention

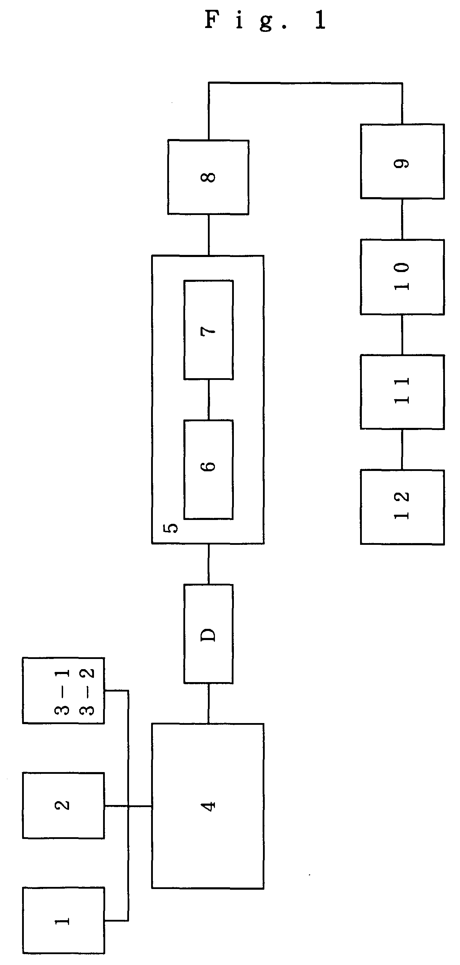

A general method of producing the cellulose sponge of the present invention will now be described with reference to the flow sheet of Fig.1.

The cellulose sponge of the present invention is prepared into cellulose sponge product 12 with no reinforcing fiber contained therein, by mixing process 4 of mixing crystal mirabilite 2 and polyol compound 3-2, and surfactant 3-1 if necessary, with viscose 1, molding process 5 in a mold, comprising a first temperature holding process 6 of heating the resulting raw sponge solution D at relatively low temperature and second temperature holding process 7 of subjecting the resulting solution to a heating process at a temperature higher than that at the first temperature holding process 6, mold releasing process 8 of releasing the product from the mold, subsequent post-treatment process 9 and drying process 10, and subsequent cutting process 11 of cutting the resulting product into a shape required for the desired product.

Viscose

As viscose 1 in the flow sheet of Fig.1, generally, use is made of viscose of a type for viscose rayon, industrially advantageously, and the viscose is effectively utilized. As the viscose for viscose rayon, use may be made of for example routine viscose and viscose for high-tenacity rayon, with no specific limitation. The polymerization degree of the cellulose component in the viscose is 200 to 400, particularly preferably 250 to 350. A cellulose with a polymerization degree higher than 400 is not preferable, because the sponge from such cellulose is hard. A cellulose with a polymerization degree lower than 200 is neither preferable, because the resulting sponge is fragile.

A type of viscose at a cellulose concentration within a range of 5 to 10 % by weight, an alkali concentration within a range of 3 to 8 %, a Hottenrote value within a range of 5 to 12, a falling ball viscosity within range of 20 to 300 sec/20 °C and a $\gamma$ value within a range of 45 to 70, is preferably used.

If a type of viscose at a cellulose concentration below 5 % by weight is used, the viscose viscosity is decreased to precipitate crystal mirabilite in the raw sponge solution (D), so that the pores in the resulting sponge are non-uniform, unpreferably. If a type of viscose at a cellulose concentration higher than 10 % by weight is used, adversely, the viscose viscosity is increased so that crystal mirabilite can hardly be mixed therein uniformly. Consequently, the pores of the cellulose sponge are too non-uniform to prepare a preferable cellulose sponge with uniform softness and uniform strength.

If the falling ball viscosity is lower than 20 sec, the viscose viscosity is too low so that crystal mirabilite eventually precipitates in the raw sponge solution (D). Hence, the pores in the sponge are non-uniform, unpreferably based on the same reason. If the viscosity is higher than 300 sec, the viscose viscosity is too high so that crystal mirabilite can hardly be uniformly mixed therein. Consequent pores in the sponge are non-uniform, unpreferably based on the same reason.

A type of viscose at an alkali concentration below 3 %, a type of viscose at a $\gamma$ value below 45, or a type of viscose at a Hottenrote value below 5, is not preferable because such viscose so readily solidifies the raw sponge solution (D) that solidification occurs during the kneading and filling procedures of crystal mirabilite into a mold. A type of viscose at an alkali concentration above 10 %, a type of viscose at a $\gamma$ value above 70, or a type of viscose at a Hottenrote value above 12, is not preferable, because such viscose hardly solidifies the raw sponge solution (D) which means to need a longer time for the solidification process 5, unpreferably.

Crystal mirabilite

Crystal mirabilite 2 in the flow sheet of Fig.1 is preferably of an average particle size 0.2 mm or less, particularly within a range of 0.01 mm to 0.2 mm. If the average particle size of crystal mirabilite 2 is larger than 0.2 mm, the diameter of the pores (cells) composing the cellulose sponge gets larger, to affect the contraction during drying and the recovery rate to the original swelling state after repeated swelling and drainning action. Furthermore, the strength of the resulting cellulose sponge is of uneven.

Furthermore, thick parts may develop in the wall forming the pores, which may deteriorate softness when the sponge is swollen in aqueous alcohol solutions. In such case, the softness of the sponge is deteriorated when immersed in aqueous alcohol solutions, which is not desirable for medical use.

Crystal mirabilite is satisfactorily added to an amount 50- to 100-fold (by weight ratio), preferably 60- to 80-fold that of the cellulose in viscose. If crystal mirabilite is added to an amount less than 50-fold that of the cellulose weight in viscose, the apparent density after drying is too elevated, which causes thick pore wall so that the resulting sponge has only deteriorated softness. If the amount is more than 100-fold, the viscosity of the raw sponge solution (D) is so high during the mixing procedure that the strength of the resulting sponge is too reduced, unpreferably.

Surfactant

Surfactant is represented by "3-1" in the flow sheet of Fig.1, which serves to improve the fluidity of the raw sponge solution (D) when added to viscose 1. In other words, surfactant serves to reduce the viscosity suitably, so that air is less incorporated during the filling of the solution into a mold thereby improving the workability to produce a cellulose sponge with stable quality such as strength and softness. Surfactant 3-1 may satisfactorily be added to the viscose 1, if necessary, and when the raw sponge solution (D) comprises viscose 1 with a relatively high alkali concentration, a relatively high $\gamma$ value and a relatively high Hottenrote value, the fluidity of the solution is so sufficiently high that not any surfactant 3-1 may necessarily be added into the solution.

As such surfactant, use may be made of anionic surfactants and/or nonionic surfactants. Cationic surfactants are not preferable, because the surfactants modify viscose. Particularly preferable surfactants include one or more selected from the group consisting of sodium dodecyl sulfate, sorbitan monooleate ester, and ethylene oxide adduct of these surfactants [for example, sorbitan monooleate ester + 20 $(C_2H_4O)$]. Any of these surfactants may preliminarily be added to viscose, for ready use. The amount thereof to be added is preferably at 1.0 % by weight or less to viscose. The amount is more preferably at 0.02 to 1.0 % by weight, particularly preferably at 0.1 to 1.0 % by weight. An amount at less than 0.02 % by weight cannot exhibit the effect even if added. The effect thereof at an amount of 1.0 % by weight or more is the same as at 0.1 to 1.0 % by weight, which is useless.

For the purpose of improving the properties and softness of cellulose sponge, conventionally, it has been known that the addition of surfactant as a dispersing agent for dispersing a reinforcing fiber and mirabilite in viscose, is effective. However, no examination has been made about the conditions for adding surfactants in terms of the properties of the resulting cellulose sponge after impregnation with aqueous alcohol solutions. By the method of producing the cellulose sponge of the present invention, a nonionic surfactant of an HLB of 9.9 or less may satisfactorily be used as well. Conventionally, reports have been issued such that when a surfactant of an HLB less than 10 is added, the resulting cellulose sponge has poor softness during drying and low recovery rate to the original swelling state after repeated swelling and drainning action (Japanese Patent Laid-open No. Hei 2-135235). However, the present inventors have made investigations to find that the properties such as fluid absorption ratio of aqueous alcohol solutions and softness in aqueous alcohol solutions have no relation with the HLB value of surfactant. In other words, even a surfactant of an HLB value less than 10 can serve to produce a cellulose sponge which can be impregnated with aqueous alcohol solutions and is suitable for medical use. This should be very notable.

Preferably, a solid surfactant preliminarily mixed with polyethylene glycol is then added, as will be described below.

Polyalkylene glycol

Polyalkylene glycol is represented by 3-2 in the flow sheet of Fig.1. For the purpose of improving the properties and softness of cellulose sponge, conventionally, it has been known that polyol compounds may satisfactorily be added to viscose. However, no examination has been made about the conditions for adding polyol compounds in terms of the properties of the resulting cellulose sponge after impregnation with aqueous alcohol solutions.

The present inventors have found that addition of polyol compounds can elevate the fluid absorption ratio of cellulose sponge in aqueous alcohol solutions. Among polyol compounds, polyoxyalkylene glycol of an average molecular weight of 100 to 500 effectively elevates the fluid absorption ratio of aqueous alcohol solutions so highly, including polyethylene glycol having an average molecular weight of preferably 200 to 500, more preferably around 400.

If the molecular weight of polyalkylene glycol is above 500, the fluid absorption ratio of aqueous alcohol solutions is elevated at some extent, but the resulting cellulose sponge is fragile with only poor strength, and additionally, the sponge causes difficulty in mold releasing. The addition of a reinforcing fiber so as to prevent this may cause lint generation during use. Preferably, polyethylene glycol is added at an amount of 2 to 4 % by weight to viscose. Below 2 % by weight, the contraction rate during drying is larger, while the fluid absorption ratio of water and aqueous ethanol solutions is reduced. Above the level, adversely, the fluid absorption ratio reaches equilibrium, with no sufficient effect of addition, economically unpreferably.

Among known polyol compounds, glycerin and the like cannot be used as such additive for viscose because the resulting sponge may get hard, but the reason is not yet elucidated.

In adding polyethylene glycol into viscose, a part of polyethylene glycol may preliminarily be added and then, the remaining part thereof together with a surfactant may be added so as to adjust the amount thereof to be added.

Mixing process

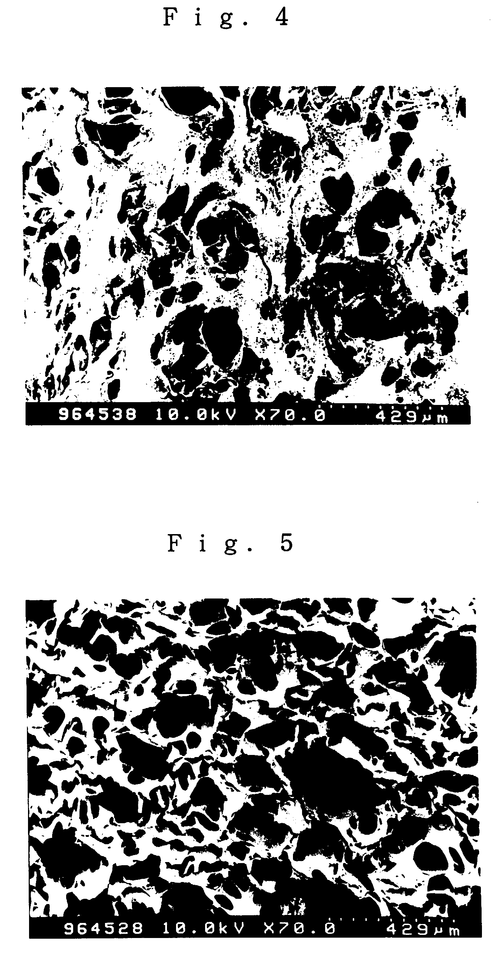

Mixing process of mixing the individual raw materials together to prepare the raw sponge material (D) is represented by 4 in the flow sheet of Fig.4. At the mixing process, generally, polyalkylene glycol is mixed with viscose, followed by addition of a surfactant if necessary, and finally, mirabilite is mixed into the resulting mixture. If necessary, further, a pigment and an X-ray non-transmitting material and the like may be mixed into the mixture at the mixing process.

Any mixing system with a cooling mechanism capable of adjusting the viscose temperature below 20 °C may be used, with no specific limitation. If the viscose temperature is above 20 °C, the viscose starts to be solidified, leading to difficulty in forming uniform pores. The three compounds, namely crystal mirabilite, polyalkylene glycol and surfactant, may be added and mixed simultaneously into viscose, but preferably, crystal mirabilite is added to viscose, into which polyalkylene glycol and surfactant preliminarily have been added.

Molding process

Molding process is represented by 5 in the flow sheet of Fig.1, and the molding process comprises filling process of filling the raw sponge solution (D) recovered from the mixing process 4 into a mold (not shown in figures), first temperature holding process 6 of holding the raw sponge solution (D) filled in the mold within a first temperature range and second temperature holding process 7 of holding the solution within a second temperature range.

a) Mold

The shape of the mold to be used at the molding process should be selected depending on the use. Raw sponge molded article of a large size or an inconstant size can be cut into given size, which is then used satisfactorily, and in that case, the properties in aqueous ethanol solutions as described above are not modified. The cellulose sponge of the present invention preferably has cut face as less as possible, to such an extent that the ratio of the area of the cut face to the total surface area is 50 % or less. If the cut face exceeds 50 % of the surface area of a molded article, unpreferably, lint is readily generated when the cellulose sponge is used clinically, particularly when the sponge is rubbed on a patient with a diseased part.

Tubular molds may be used, and the inner shape of the molds may satisfactorily be either of cylindrical shape or columnar shape. From the respect of the sponge shape for medical use, however, cylindrical shape is preferable. The inner diameter of a tubular mold is 5 mm to 200 mm, taking into account the sponge size to be applied to medical use, and the molded article just as released from the mold can produce a columnar sponge of a suitable diameter size with good handleability. Molded columnar sponge may satisfactorily be cut into a suitable length vertical to the axial direction, for use. By adopting the sponge size after mold release along the axial direction as the size for use, to prepare a sponge with no cut surface on the side faces and both the ends after sponge molding, lint generation is effectively prevented.

The material of a mold, essentially having a heat resistant temperature above 90 °C, may satisfactorily be used. For example, the material is plastic, glass, a material coated with ceramic and plastic and the like. More specifically, the plastics include for example PTFE, polypropylene, heat-resistant vinyl chloride, high-density polyethylene and the like, preferably, because they are relatively less expensive. These molding materials have better sponge releasability compared with that of metals, and hence, sponges with good appearance can be released even if no releasing agent is preliminarily coated thereon. No concern of the residue of releasing agents on the surface of sponges arises. Thus, the problem of the remaining releasing agents can be overcome, which is a concern for the use of the sponge for disinfection.

b) Filling process (not shown in figures)

At the molding process, care should be taken when filling the raw sponge solution (D) into a mold so as to prevent air incorporation, in such a manner that filling should be done in a mold at a state under reduced pressure. In filling the raw sponge solution (D) into a tubular mold, the two ends of the tubular mold should be arranged at different heights, to fill the solution from the bottom thereby eliminating air incorporation.

c) First temperature holding process (low-temperature solidification process)

In accordance with the present invention, importantly, the raw sponge solution filled in a mold at the filling process of the molding process is subjected to the temperature holding process within a range of 35 °C or more to 70 °C or less (first temperature range) for 30 minutes or more to 24 hours or less. Particularly preferably, the process is carried out by holding the solution at 35 to 45 °C for 60 minutes to 5 hours. More specifically, the solution should be held within the first temperature range, until the fluidity of the raw sponge solution is eliminated totally.

If the first temperature range is below 35 °C, the holding process takes a too long term, which is not practical industrially. If the heating process above 70 °C at the solidification process conventionally carried out for producing cellulose sponge is effected at the first temperature holding process, gaseous carbon disulfide evolves rapidly to generate non-uniform pores in the resulting sponge, thereby leading to non-uniform softness. If the first temperature range is above 70 °C, additionally, the sponge solution is solidified starting from the periphery, so that hard crust develops at parts in contact to the mold. Thus, the strength of the periphery of the resulting cellulose sponge is different from the strength of the inside thereof, which is not suitable for medical use.

d) Second temperature holding process (high-temperature solidification process)

The solution can absolutely be solidified at the first temperature holding process, but the process requires a long time for solidification, industrially inconveniently. Immediately following the first temperature holding process, an additional second temperature holding process of holding the solution at a high temperature above 90 °C (second temperature range) within a range of 30 minutes to 5 hours is therefore an industrially advantageous means for shortening the solidification time. More preferably, the range is at a temperature of 90 °C or more to 100 °C or less for one hour to 4 hours. If the second temperature range is below 90 °C, the resulting sponge can hardly maintain any constant shape after mold releasing. Consequently, the alcohol fluid absorption ratio and the strength at its wet state have larger variations, so that the overall properties of the resulting sponge are deteriorated for medical use.

If the heating process at the second temperature holding process is incomplete, non-solidified raw sponge solution may be soluble during washing in water, to cause insufficient formation of sponge.

If the second temperature holding process is carried out without passing through the thermal hysteresis at the low temperature at the first temperature holding process, rapidly evolving gaseous carbon disulfide may block the formation of uniform pores. As has been described above, the resulting cellulose sponge has thicker pore wall if the first temperature holding process of the present invention is skipped, so that the formed pore wall is not uniform but thick. Such part gets hard on contact to aqueous alcohol solutions, thus suppressing the softness when the part absorbs aqueous alcohol solutions.

The first and second temperature holding processes may be carried out with no specific limitation, as long as the processes can hold the temperatures uniformly. For industrially uniform practice of the thermal treatment, advantageously, the raw sponge solution as is placed in a mold is charged into warm water for carrying out the processes.

Because the raw sponge solution is subjected to thermal treatment at the first and second temperature holding processes, carbon disulfide gas evolves. So as to prevent gaseous residue, therefore, means such as gas discharge outlet should be arranged on a mold to prepare the mold from which such gas can be removed. If gas withdrawal is insufficiently carried out, giant pores may generate other than pores with a uniform pore diameter in the resulting cellulose sponge after washing off crystal mirabilite at the washing process in water. Thus, non-uniform pores may develop in the cellulose sponge, and additionally, the pore wall forming pores is not uniform, so that the strength is not uniform, unpreferably, leading to the generation of portions with mechanically weak strength.

The presence of pores of such non-uniform sizes may cause insufficient absorption of liquids when the sponge absorbs the liquids, or may cause additionally the hardening of the portions with thicker pore wall during the absorption of aqueous alcohol solutions, thereby deteriorating the softness of the cellulose sponge. More specifically, for the purpose of producing the cellulose sponge of the present invention, importantly, only micro-pores derived from the solubilization of crystal mirabilite under control of the particle diameter should be formed.

When the molding process is carried out at a state before the gas is eliminated, the inner pressure is raised to prepare very hard cellulose sponge, unpreferably, if such state is retained during the solidification. In other words, it is important that the first and second temperature holding processes at the molding process should be carried out under no pressure applied.

## Mold releasing process

Mold releasing process is represented by 8 in the flow sheet of Fig.1. Mold releasing may readily be carried out because the raw sponge solution contracts during solidification, but it should be noted that deformation or narrowing procedures at that state may be memorized as the shapes then.

## Post-treatment process

Post-treatment process is represented by 9 in the flow sheet of Fig.1. The post-treatment process comprises water washing process, acid regeneration process, desulfurization process, bleaching process and neutralization process, and well known processes may be selected therefor.

### a) Water washing process

The molded article from the mold releasing process is washed in hot water until the crystal mirabilite added into the raw sponge solution is completely solubilized. Then, water washing at ambient temperature should preferably be conducted. Via the water washing, surfactants therein are removed together with mirabilite. If mirabilite and surfactants may remain, the resulting article is not suitable for medical use.

### b) Acid washing process (cellulose regeneration process)

The process is carried out by the same method as in the regeneration process of routine viscose rayon; for example, the molded article after the water washing process is immersed in an aqueous sulfuric acid solution at a concentration of about 40 to 100 g/l. Via the process, viscose is completely regenerated into cellulose. The aqueous sulfuric acid solution to be used is particularly preferably at a concentration range of 50 to 90 g/l. If the concentration of the aqueous sulfuric acid solution is higher than 100 g/l, the sponge surface may be rough sometimes. If the concentration is below 40 g/l, the regeneration proceeds slowly. The immersion time is preferably 2 to 30 minutes. For a term shorter than 2 minutes, the cellulose cannot completely be regenerated. Above 30 minutes, regeneration is already in completion before the term is out, so no progress of regeneration is observed. The temperature is preferably 20 to 50 °C. Below 20 °C, regeneration gets slow, unpreferably; above 50 °C, the sponge surface may eventually turn rough, unpreferably.

### c) Desulfurization process (alkali washing process)

At the desulfurization process, the molded article after the acid regeneration process is treated with an aqueous sodium hydroxide solution or with an aqueous sodium sulfide solution. Via the process, the sulfur residue can be reduced in the resulting sponge. The process comprises immersion in a mixture solution of an aqueous sodium hydroxide solution at a concentration of 1 to 4 g/l and an aqueous sodium sulfide solution at a concentration of 0.5 to 2 g/l. When the aqueous sodium hydroxide solution is at a concentration below 1 g/l or the aqueous sodium sulfide solution is at a concentration below 0.5 g/l, no such effect can be brought about. When the aqueous sodium hydroxide solution is at a concentration above 4 g/l or the aqueous sodium sulfide solution is at a concentration above 2 g/l, the sponge surface may occasionally turn rough. The temperature of the immersing solution is preferably 40 to 80 °C. The immersion time is preferably 10 to 60 minutes. If the temperature is below 40 °C or if the immersing time is shorter than 10 minutes, the effect is hardly brought about. Alternatively, when the temperature is higher than 80 °C or when the immersion time is longer than 60 minutes, the sponge surface may sometimes turn rough.

More specifically, the process is carried out by immersing the article in a mixture solution of an aqueous sodium hydroxide solution at a concentration of 1 to 4 g/l and an aqueous sodium sulfide solution at a concentration of 0.5 to 2 g/l at 40 to 80 °C for 10 to 60 minutes.

d) Bleaching process

At the bleaching process, the resulting article is satisfactorily immersed in an aqueous sodium hypochlorite solution at a concentration below 0.1 to 3 g/l or an aqueous hydrogen peroxide solution at ambient temperature for 10 to 30 minutes. No bleaching effect can be realized at a concentration of 0.1 g/l of an aqueous sodium hypochlorite solution or hydrogen peroxide. If the concentration of an aqueous sodium hypochlorite solution or hydrogen peroxide is higher than 3 g/l, the sponge surface may be rough occasionally. The temperature of the immersing solution is preferably 20 to 50°C. The immersion time is preferably 10 to 30 minutes. If the temperature is below 20 °C or if the immersing time is shorter than 10 minutes, the bleaching effect is hardly brought about. Alternatively, when the temperature is higher than 50 °C or when the immersion time is longer than 30 minutes, the sponge surface may sometimes turn rough.

e) Neutralization process

At the neutralization process, the chemical reagents used at the bleaching process should be washed off in water so as to avoid the residue thereof.

Drying process

Drying process is represented by 10 in the flow sheet of Fig.1. A variety of known means can be used as the drying means.

Cutting process

Cutting process is represented by 11 in the flow sheet of Fig.1. However, no such cutting process is necessary, when the shape of the resulting cellulose sponge of itself is used for product.

Because the molded article through the drying process is cut into a desirable shape for the objective cellulose sponge product, the cellulose sponge for the purpose of medical use should have cut face as less as possible, so as to avoid lint generation, as has been described above. Thus, the ratio of the area of the cut face occupying the total surface area should be within 50 %. Both the ends of the cellulose sponge molded by using a columnar mold is cut vertically along the axial direction into the shape of the objective cellulose sponge product, but cutting should be carried out importantly so as to satisfy the relation represented by the formula 1 to suppress lint generation.

Cellulose sponge product

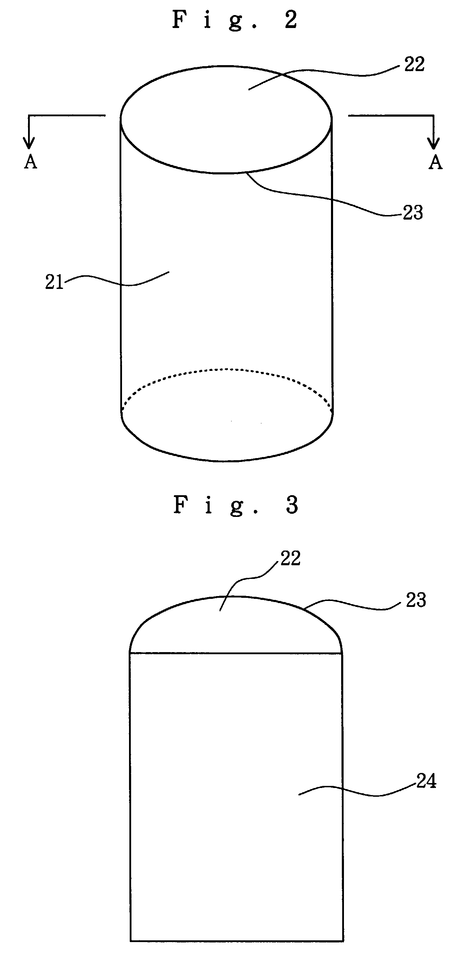

The cellulose sponge product of the present invention is represented by 12 in the flow sheet of Fig.1. Fig.2 depicts a cylindrical product as one embodiment shape of the cellulose sponge product of the present invention. In Fig.2, 21 represents peripheral surface in contact to the mold; 22 represents the cross section after vertical cutting along the axial direction; and 23 represents the ridgeline as the border line between the peripheral surface 21 and the cross section 22. Fig.3 depicts the state of the sponge cut along the direction A-A (axial direction); and 24 represents the longitudinal face thereof.

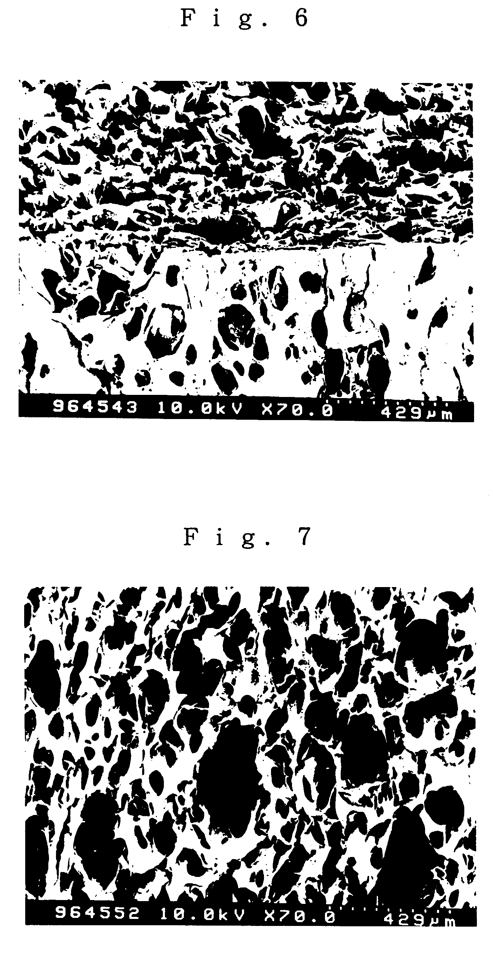

Figs.4 to 7 depict the scanning electron microscopic photographs of the surface state of the cellulose sponge of the present invention, at 70 magnification. The scanning electron microscopic photograph of Fig.4 depicts the peripheral surface 21 of Fig.2; the scanning electron microscopic photograph of Fig.5 depicts the cross section 22 of Fig.2; the scanning electron microscopic photograph of Fig.6 depicts the ridgeline 23 of Fig.2; and the scanning electron microscopic photograph of Fig.7 depicts the longitudinal face 24 of Fig.3.

In the scanning electron microscopic photograph of Fig.4 depicting the peripheral surface 21 of Fig.2, it is shown that the outer pore wall of the cellulose sponge is on a plane parallel to the peripheral surface 21. On the contrary, the scanning electron microscopic photograph of Fig.5 depicting the cross section 22 formed through cutting and the photograph of Fig.7 depicting the longitudinal face 24 of Fig.7, both the photographs of the cut face 24 show that the pore wall is in the vertical direction to the cut face, and therefore, it is assumed that if such cut face touches and rubs the diseased part of a patient, the face causes the generation of lint in the form of threads. Thus, the area of the cut face of the cellulose sponge for medical use should be as less as possible, and the cellulose sponge in the cylindrical shape as shown in Fig.2 is preferable because the cut face is less.

When conventional cellulose sponges containing reinforcing fibers are cut out from a large cellulose sponge mass, the reinforcing fibers are modified into short fibers, which are readily dissociated in a lint shape from the cross section of the pore wall. Because the cellulose sponge of the present invention does not contain any reinforcing fiber, on contrast, no lint due to the presence of any reinforcing fiber is observed in the scanning electron microscopic photographs of Figs.4 to 7.

It is observed in the scanning electron microscopic photographs of Figs.4 to 7 that any thick part as is observed in the conventional cellulose sponges is present in the partition wall forming the pores and that extremely thin partition wall forms the pores. Such structure may possibly contribute to the exertion of one of the significant properties of the cellulose sponge of the present invention, namely the property in aqueous alcohol solutions.

Through the aforementioned processes, the cellulose sponge, with a uniform structure at an apparent dry density of 0.01 to 0.07 g/ml and a tensile strength of 8 to 25 $g/mm^2$ when wet in water even without any reinforcing fiber contained therein, with suitable softness even immersed in alcohol and with a fluid absorption ratio of aqueous alcohol solutions being 1500 % or more, can be recovered in accordance with the present invention.

The present invention will now be described in detail in examples, which are only illustrated, not by way of limiting. Herein, the density and softness are measured as described below.

Density: a 1-$cm^3$-volume sample was sectioned from an optional part of a sponge dried by a dryer, and the weight was determined.

Softness: functional test of a sponge after drying was performed by ten females, and the results were assessed on the basis of the following four ranks.

◎ ---- very good
○ ---- good
△ ---- normal
X ---- poor

Example 1

Into a type of viscose, containing cellulose of a polymerization degree of 320 at 7 % by weight and an alkali at 6 % by weight were added and mixed polyethylene glycol of an average molecular weight of 400 at 3 % by weight to the viscose, sodium dodecyl sulfate at 0.4 % by weight to the viscose and crystal mirabilite of an average particle diameter of 0.12 mm at a weight 65-fold to the weight of the cellulose, to prepare a raw sponge solution.

A heating process was carried out, comprising charging the raw sponge solution into a tubular container made of a heat-resistant polyvinyl chloride and equipped with a gas discharge outlet of an inner size of 200 mm × 200 mm × 100 mm, and holding the solution in a water tank at 40 °C for 60 minutes, and then holding the solution in a water tank at 90 °C for 120 minutes. As the outcome of the process, the raw sponge solution was solidified, and even after releasing the resulting molded article from the mold, the article could retain its shape.

Removing mirabilite in boiling water at 90 °C or more and immersing the resulting intermediate sponge in an aqueous 50 g/l sulfuric acid solution for 120 minutes, the cellulose was regenerated. Furthermore, the regenerated cellulose was immersed in a mixture solution of an aqueous 2.5 g/l caustic soda solution and an aqueous 1.3 g/l sodium sulfate solution at 50 °C for 10 minutes, for treatment for desulfurization. Subsequently, the resulting product was immersed in an aqueous 0.2 g/l sodium hypochlorite solution at ambient temperature for 10 minutes for desulfurization and bleaching treatment, prior to washing in water. Then, the product was dried to recover the cellulose sponge of Example 1. The performance of the resulting cellulose sponge matrix was assessed by the predetermined methods. The results are shown in Table 1 below.

Example 2

The same procedures as in Example 1 were conducted, except that sodium dodecyl sulfate was not used and crystal mirabilite was added at a weight 75-fold to the weight of the cellulose, at the process of producing the cellulose sponge of Example 1, to produce the cellulose sponge of Example 2. The performance of the resulting cellulose sponge mass was assessed by the same method as in Example 1. The results are shown in Table 1 below.

Example 3

Except for the use of the same raw sponge solution as in Example 1 and that the retention time at the first temperature holding process was set at 1,440 minutes (24 hours) at the process of producing the cellulose sponge of Example 1, the same procedures as in Example 1 were followed to recover the cellulose sponge of Example 3. The performance of the resulting cellulose sponge mass was assessed by the same method as in Example 1. The results are shown in Table 1 below.

Example 4

The same procedures as in Example 1 were conducted, except that crystal mirabilite was added at a weight 75-fold to the weight of the cellulose and the retention time at the first temperature holding process was set at 120 minutes at the processes of producing the cellulose sponge of Example 1, to produce the cellulose sponge of Example 4. The performance of the resulting cellulose sponge mass was assessed by the same method as in Example 1. The results are shown in Table 1 below.

Example 5

Except for the use of a surfactant polyoxyethylene cetyl ether (nonion P-213) of an HLB of 15.7 in place of sodium dodecyl sulfate (SDS) of an HLB value of 40 at the process of producing the cellulose sponge in Example 1, the same procedures in Example 1 were followed to recover the cellulose sponge of Example 5. The performance of the resulting cellulose sponge mass was assessed by the same method as in Example 1. The results are shown in Table 1 below.

Example 6

Except for the use of a surfactant polyoxyethylene cetyl ether (nonion K-230) of an HLB of 9.5 in place of sodium dodecyl sulfate (SDS) of an HLB value of 40 at the process of producing the cellulose sponge in Example 1, the same procedures in Example 1 were followed to recover the cellulose sponge of Example 6. The performance of the resulting cellulose sponge mass was assessed by the same method as in Example 1. The results are shown in Table 1 below.

Example 7

Except for the use of a surfactant sorbitan monooleate ester EO20 adduct (Tween 80) of an HLB of 15.0 in place of sodium dodecyl sulfate (SDS) of an HLB value of 40 at the process of producing the cellulose sponge in Example 1, the same procedures in Example 1 were followed to recover the cellulose sponge of Example 7. The performance of the resulting cellulose sponge mass was assessed by the same method as in Example 1. The results are shown in Table 1 below.

Example 8

Except for the use of a surfactant sorbitan monolaurate ester (Span 20) of an HLB of 8.6 in place of sodium dodecyl sulfate (SDS) of an HLB value of 40 at the process of producing the cellulose sponge in Example 1, the same procedures in Example 1 were followed to recover the cellulose sponge of Example 8. The performance of the resulting cellulose sponge mass was assessed by the same method as in Example 1. The results are shown in Table 1 below.

Example 9

Except for the use of a surfactant sorbitan monooleate ester (Span 80) of an HLB of 4.3 in place of sodium dodecyl sulfate (SDS) of an HLB value of 40 at the process of producing the cellulose sponge in Example 1, the same procedures in Example 1 were followed to recover the cellulose sponge of Example 9. The performance of the resulting cellulose sponge mass was assessed by the same method as in Example 1. The results are shown in Table 1 below.

Example 10

Except for the use of a surfactant sorbitan monooleate ester (Span 80) of an HLB of 1.8 in place of sodium dodecyl sulfate (SDS) of an HLB value of 40 at the process of producing the cellulose sponge in Example 1, the same procedures in Example 1 were followed to recover the cellulose sponge of Example 10. The performance of the resulting cellulose sponge mass was assessed by the same method as in Example 1. The results are shown in Table 1 below.

Comparative Example 1

Except for the use of viscose containing cellulose of a polymerization degree of 170 in place of viscose containing cellulose of a polymerization degree of 320 at the process of producing the cellulose sponge in Example 1, the same procedures in Example 1 were followed to recover the cellulose sponge of the present Comparative Example 1. The performance of the resulting cellulose sponge mass was assessed by the same method as in Example 1. The results are shown in Table 2 below.

Comparative Example 2

Except for the use of viscose containing cellulose of a polymerization degree of 500 in place of viscose containing cellulose of a polymerization degree of 320 at the process of producing the cellulose sponge in Example 1, the same procedures in Example 1 were followed to recover the cellulose sponge of the present Comparative Example 2. The performance of the resulting cellulose sponge mass was assessed by the same method as in Example 1. The results are shown in Table 2 below.

Comparative Example 3

Except for the use of polyethylene glycol (PEG) of a molecular weight of 600 in place of polyethylene glycol (PEG) of a molecular weight of 400 at the process of producing the cellulose sponge in Example 1, the same procedures in Example 1 were followed to recover the cellulose sponge of the present Comparative Example 3. The performance of the resulting cellulose sponge mass was assessed by the same method as in Example 1. The results are shown in Table 2 below.

Comparative Example 4

Except for the use of glycerin of a molecular weight of 68 in place of polyethylene glycol (PEG) of a molecular weight of 400 at the process of producing the cellulose sponge in Example 1, the same procedures in Example 1 were followed to recover the cellulose sponge of the present Comparative Example 4. The performance of the resulting cellulose sponge mass was assessed by the same method as in Example 1. The results are shown in Table 2 below.

Comparative Example 5

Except for the use of polyethylene glycol (PEG) of a molecular weight of 2,000 in place of polyethylene glycol (PEG) of a molecular weight of 400 at the process of producing the cellulose sponge in Example 1, the same procedures in Example 1 were followed to recover the cellulose sponge of the present Comparative Example 5. The performance of the resulting cellulose sponge mass was assessed by the same method as in Example 1. The results are shown in Table 2 below.

Comparative Example 6

Except that polyethylene glycol (PEG) of a molecular weight of 400 was added at an amount of 10 % by weight to viscose instead of 3 % by weight to viscose at the process of producing the cellulose sponge in Example 1, the same procedures in Example 1 were followed to recover the cellulose sponge of the present Comparative Example 6. The performance of the resulting cellulose sponge mass was assessed by the same method as in Example 1. The results are shown in Table 2 below.

Comparative Example 7

Except that polyethylene glycol (PEG) of a molecular weight of 400 was added at an amount of 0.2 % by weight to viscose instead of 3 % by weight to viscose at the process of producing the cellulose sponge in Example 1, the same procedures in Example 1 were followed to recover the cellulose sponge of the present Comparative Example 7. The performance of the resulting cellulose sponge mass was assessed by the same method as in Example 1. The results are shown in Table 2 below.

Comparative Example 8

Except that the retention conditions at the temperature holding process was modified as 90 °C and 120 minutes and the second temperature holding process was not done at the process of producing the cellulose sponge in Example 1, the same procedures in Example 1 were followed to recover the cellulose sponge of the present Comparative Example 8. The performance of the resulting cellulose sponge mass was assessed by the same method as in Example 1. The results are shown in Table 2 below.

Comparative Example 9

Except that the retention conditions at the temperature holding process was modified as 75 °C and 120 minutes and the second temperature holding process was not done at the process of producing the cellulose sponge in Example 1, the same procedures in Example 1 were followed to recover the cellulose sponge of the present Comparative Example 9. The performance of the resulting cellulose sponge mass was assessed by the same method as in Example 1. The results are shown in Table 2 below.

Comparative Example 10

Except that the average particle diameter of crystal mirabilite was 3 mm instead of 0.12 mm at the process of producing the cellulose sponge in Example 1, the same procedures in Example 1 were followed to recover the cellulose sponge of the present Comparative Example 10. The performance of the resulting cellulose sponge mass was assessed by the same method as in Example 1. The results are shown in Table 2 below.

Comparative Example 11

Except that crystal mirabilite was added at a weight 20-fold to the weight of the cellulose at the process of producing the cellulose sponge in Example 1, the same procedures in Example 1 were all followed to recover the cellulose sponge of the present Comparative Example 11. The performance of the resulting cellulose sponge mass was assessed by the same method as in Example 1. The results are shown in Table 2 below.

Table 1

| Example No. | Example 1 | Example 2 | Example 3 | Example 4 | Example 5 | Example 6 | Example 7 | Example 8 | Example 9 | Example 10 |
|---|---|---|---|---|---|---|---|---|---|---|
| **Sponge production conditions** | | | | | | | | | | |
| polymerization degree of cellulose in viscose | 320 | 320 | 320 | 320 | 320 | 320 | 320 | 320 | 320 | 320 |
| polyol compound — molecular weight of polyol compound | PEG 400 | PEG 400 | PEG 400 | PEG 400 | PEG 400 | PEG 400 | PEG 400 | PEG 400 | PEG 400 | PEG 400 |
| amount added (% by weight to viscose) | 3 | 3 | 3 | 3 | 3 | 3 | 3 | 3 | 3 | 3 |
| surfactant | SDS | none | SDS | SDS | nonion P | nonion K | Tween | Span20 | Span80 | Span85 |
| HLB | 40 | | 40 | 40 | 15.7 | 9.5 | 15.0 | 8.6 | 4.3 | 1.8 |
| amount added (% by weight to viscose) | 0.4 | | 0.4 | 0.4 | 0.4 | 0.4 | 0.4 | 0.4 | 0.4 | 0.4 |
| average particle size of crystal mirabilite (mm) | 0.12 | 0.12 | 0.12 | 0.12 | 0.12 | 0.12 | 0.12 | 0.12 | 0.12 | 0.12 |
| amount added (ratio in weight to cellulose weight) | 65 | 75 | 65 | 75 | 65 | 65 | 65 | 65 | 65 | 65 |
| temperature for first temperature holding process (°C) | 40 | 40 | 40 | 40 | 40 | 40 | 40 | 40 | 40 | 40 |
| time (minute) | 60 | 60 | 1440 | 120 | 60 | 60 | 60 | 60 | 60 | 60 |
| temperature for second temperature holding process (°C) | 90 | 90 | 90 | 90 | 90 | 90 | 90 | 90 | 90 | 90 |
| time (minute) | 120 | 120 | 120 | 120 | 120 | 120 | 120 | 120 | 120 | 120 |
| **Outcome of performance assessment** | | | | | | | | | | |
| apparent density during drying (g/mℓ) | 0.05 | 0.06 | 0.06 | 0.05 | 0.03 | 0.04 | 0.05 | 0.05 | 0.06 | 0.08 |
| fluid absorption ratio of aqueous ethanol (70vol.%) solution (%) | 1970 | 1900 | 1970 | 2020 | 2800 | 2560 | 2210 | 2080 | 1680 | 1510 |
| strength when wet (g/mm²) lengthwise | 15.4 | 12.8 | 11.8 | 12.8 | 6.7 | 7.0 | 11.3 | 5.7 | 15.3 | 14.8 |
| crosswise | 10.3 | 10.5 | 9.9 | 10.5 | 5.4 | 7.2 | 10.4 | 3.5 | 11.9 | 9.6 |
| softness when wet in ethanol | ◎ | ◎ | ◎ | ◎ | ◎ | ◎ | ◎ | ◎ | ◎ | ◎ |

Table 2

| | 1 | 2 | 3 | 4 | 5 | 6 | 7 | 8 | 9 | 10 | 11 |
|---|---|---|---|---|---|---|---|---|---|---|---|
| **Sponge production conditions** | | | | | | | | | | | |
| polymerization degree of cellulose in viscose | 170 | 500 | 320 | 320 | 320 | 320 | 320 | 320 | 320 | 320 | 320 |
| polyol compound: molecular weight of polyol compound | PEG 400 | PEG 400 | PEG 600 | glycerin 68 | PEG 2000 | PEG 400 | PEG 400 | PEG 400 | PEG 400 | PEG 400 | PEG 400 |
| amount added (% by weight to viscose) | 3 | 3 | 3 | 3 | 3 | 10 | 0.2 | 3 | 3 | 3 | 3 |
| surfactant | SDS | SDS | SDS | SDS | SDS | SDS | SDS | SDS | SDS | SDS | SDS |
| HLB | 40 | 40 | 40 | 40 | 40 | 40 | 40 | 40 | 40 | 40 | 40 |
| amount added (% by weight to viscose) | 0.4 | 0.4 | 0.4 | 0.4 | 0.4 | 0.4 | 0.4 | 0.4 | 0.4 | 0.4 | 0.4 |
| average particle size of crystal mirabilite (mm) | 0.12 | 0.12 | 0.12 | 0.12 | 0.12 | 0.12 | 0.12 | 0.12 | 0.12 | 3 | 0.12 |
| amount added (ratio in weight to cellulose weight) | 65 | 65 | 65 | 65 | 65 | 65 | 65 | 65 | 65 | 65 | 20 |
| temperature for first temperature holding process (°C) | 40 | 40 | 40 | 40 | 40 | 40 | 40 | 90 | 70 | 40 | 40 |
| time (minute) | 60 | 60 | 60 | 60 | 60 | 60 | 60 | 120 | 120 | 60 | 60 |
| temperature for second temperature holding process (°C) | 90 | 90 | 90 | 90 | 90 | 90 | 90 | none | 90 | 90 | 90 |
| time (minute) | 120 | 120 | 120 | 120 | 120 | 120 | 120 | | 120 | 120 | 120 |
| **Outcome of performance assessment** | | | | | | | | | | | |
| apparent density during drying (g/ml) | 0.06 | 0.26 | 0.07 | 0.16 | — | 0.06 | 0.08 | 0.07 | 0.08 | 0.08 | 0.7 |
| fluid absorption ratio of aqueous ethanol(70vol.%)solution (%) | 600 | 385 | 1720 | 220 | — | 1940 | 1320 | 1470 | 1060 | 820 | 170 |
| strength when wet (g/mm²) lengthwise | 4.3 | 10.0 | 3.5 | 2.7 | — | 11.5 | 7.3 | 9.1 | 8.2 | 5.4 | 15.3 |
| crosswise | 3.2 | — | 2.3 | 2.4 | — | 9.7 | 7.0 | 7.2 | 5.3 | 7.3 | 16.4 |
| softness when wet in ethanol | × | × | × | × | — | ○ | × | ○ | △ | △ | × |

On comparison of the results in Tables 1 and 2, it is indicated that the cellulose sponges through the first temperature holding process within a temperature range of 35 °C or more to 70 °C or less for 30 minutes or more to 24 hours or less and the second temperature holding process within a temperature range of 90 °C or more to 100 °C or less following the first temperature holding process, in accordance with the present invention, could maintain suitable softness even when wet in ethanol, and the resulting cellulose sponges are very excellent.

According to the outcome shown in Table 1, the cellulose sponges of the present invention can maintain suitable softness even when wet in ethanol, irrespective of the HLB value of a surfactant used, so the resulting sponges are very excellent.

Example 11

The same procedures as in Example 1 were all carried out at the process of producing the cellulose sponge in Example 1, except that the retention time at the first temperature holding process was 120 minutes instead of 60 minutes, to recover the cellulose sponge of Example 11. The performance of the resulting cellulose sponge mass was assessed by the same method as in Example 1. The results are shown in Table 3 below.

Comparative Example 12

The same procedures as in Example 11 were all carried out at the process of producing the cellulose sponge in Example 11, except for the use of a polyol compound, i.e. diol-type polypropylene of a molecular weight of 400 at an addition amount of 3 % by weight to viscose in place of polyethylene glycol, to recover the cellulose sponge of Comparative Example 12. The performance of the resulting cellulose sponge mass was assessed by the same method as in Example 1. The results are shown in Table 3 below.

Comparative Example 13

The same procedures as in Example 11 were all carried out at the process of producing the cellulose sponge in Example 11, except for the use of a polyol compound, i.e. diol-type polypropylene of a molecular weight of 400 at an addition amount of 0.4 % by weight to viscose in place of polyethylene glycol, to recover the cellulose sponge of Comparative Example 13. The performance of the resulting cellulose sponge mass was assessed by the same method as in Example 1. The results are shown in Table 3 below.

Comparative Example 14

The same procedures as in Example 11 were all carried out at the process of producing the cellulose sponge in Example 11, except for the use of a polyol compound, i.e. triol-type polypropylene of a molecular weight of 300 at an addition amount of 0.4 % by weight to viscose in place of polyethylene glycol, to recover the cellulose sponge of Comparative Example 14. The performance of the resulting cellulose sponge mass was assessed by the same method as in Example 1. The results are shown in Table 3 below.

Table 3

| Example and Comparative Example No. | | Example 11 | Comparative Example 12 | Comparative Example 13 | Comparative Example 14 |
|---|---|---|---|---|---|
| Sponge production conditions | polyol compound | PEG | PPG*¹ | PPG*¹ | PPG*² |
| | molecular weight of polyol compound | 400 | 400 | 400 | 300 |
| | amount added ( % by weight to viscose) | 3 | 3 | 0.4 | 0.4 |
| | SDS addition ratio ( % by weight to viscose) | 0.4 | 0.4 | 0.4 | 0.4 |
| | average particle size of crystal mirabilite (mm) | 0.12 | 0.12 | 0.12 | 0.12 |
| | amount added (ratio in weight to cellulose weight) | 65 | 65 | 65 | 65 |
| | temperature for first temperature holding process (°C) | 40 | 40 | 40 | 40 |
| | time (minute) | 120 | 120 | 120 | 120 |
| | temperature for second temperature holding process (°C) | 90 | 90 | 90 | 90 |
| | time (minute) | 120 | 120 | 120 | 120 |
| Outcome of assessment of performance | apparent density during drying (g/mℓ) | 0.05 | 0.14 | 0.14 | 0.11 |
| | fluid absorption ratio of aqueous ethanol (70vol.%) solution (%) | 2020 | 665 | 702 | 860 |
| | strength when wet (g/mm²) lengthwise | 12.8 | 7.1 | 5.1 | 6.2 |
| | crosswise | 10.5 | 5.1 | 4.7 | 5.8 |
| | softness when wet in ethanol | ◎ | × | × | × |
| | volume contraction rate during drying (%) | 44 | 63 | 71 | 69 |

*1 : diol type      *2 : triol type

Table 3 shows that the cellulose sponges produced by using polyethylene glycol as a polyol compound by the method of the present invention have lower volume contraction rate during drying and lower apparent densities, compared with those produced by using polypropylene glycol, so these sponges can retain suitable softness even when wet in an aqueous 70 vol. % ethanol solution, having higher fluid absorption ratios in an aqueous 70 vol. % ethanol solution and greater strength, which indicates that excellent cellulose sponges are thus produced.

Industrial Applicability

The cellulose sponge of the present invention is very preferable for medical use, because the sponge still can keep superior softness even after moistened in an aqueous disinfecting alcohol solution and has a higher fluid absorption ratio of aqueous alcohol solutions of 1,500 % or more of the weight of the sponge.

Since the cellulose sponge of the present invention does not contain any reinforcing fiber, no lint generation substantially occurs due to the presence of reinforcing fibers; the cellulose sponge is preferable as a disinfecting and fluid absorbing material in direct contact to human diseased parts, for example for disinfection of cut and abrasion and disinfection of a diseased part prior to surgery, as fluid absorbing materials during surgery, and for disinfection of wounds after surgery and disinfection of mucous membranes of eye and vagina.

Because the cellulose sponge of the present invention does not contain any reinforcing fiber, the sponge is of a uniform structure and has uniform softness. More specifically, for producing conventional cellulose sponges, a raw sponge solution containing a reinforcing fiber is charged in a mold. Then, the viscosity of the raw sponge solution is increased because the reinforcing fiber is contained therein, so that the fluidity of the raw sponge solution is restricted to block the pore uniformity and the uniform thickness of the pore wall, additionally leading to a tendency of thicker pore wall. In accordance with the present invention, however, the solution does not contain any reinforcing fiber, so the resulting sponge is of a uniform structure, with the resultant excellent softness.

It is confirmed under microscopic observation that pores are formed around an extremely thin partition wall in the cellulose sponge of the present invention and the wall thickness is uniform, characteristically with no thick part therein as is observed in the conventional cellulose sponges.

The method of producing the cellulose sponge in accordance with the present invention comprises a temperature holding process within a temperature range of 35 °C or more to 75 °C or less for 30 minutes or more to 24 hours or less at the molding process and a subsequent complete solidification process, and therefore, no carbon disulfide gas rapidly evolves. Thus, uniform and micro pores are formed in the resulting cellulose sponge, so the density variation between the central part and the outer periphery hardly develops. Therefore, the cellulose sponge can procure soft touch, good recovery rate to the original swelling state after repeated swelling and drainning action in water or alcohol, sufficient mechanical strength even without any reinforcing fiber contained therein, suppressed contraction after heating and solidification during the production process and less contraction of the sponge after drying, and therefore, the sponge has smooth surface with a lower density, and has soft touch when ethanol of about 70 % is absorbed into the sponge.

By selecting a molding method of the cellulose sponge of the present invention so that the area of the cut face of the sponge to the total surface area might be 50 % or less, or by specifying the cellulose sponge as being of a columnar body, having the cut face almost vertical to the axial direction and setting the ratio L/D of the length L in the axial direction to the diameter D of the columnar body at 0.5 to 2, the molded article has less cut face and can thus prevent lint generation due to the presence of the cut face therein.

**Claims**

1. A cellulose sponge comprising substantial regenerated cellulose without any reinforcing fiber contained therein, and having the following characteristic properties;

   (1) the fluid absorption ratio of aqueous alcohol solutions should be within 1,500 % or more to 3,000 % or less; and
   (2) the apparent density during drying should be within 0.01 g/ml or more to 0.07 g/ml or less.

2. A cellulose sponge according to claim 1, wherein the strength when wet in water is 8 g/mm$^2$ or more to 25 g/mm$^2$ or less.

3. A cellulose sponge according to claim 1 or 2, wherein the ratio of the area of the cut face to the total surface area is 50 % or less.

4. A cellulose sponge of a columnar body for medical use, having the cut face almost vertical to the axial direction wherein the ratio L/D of the length L in the axial direction to the diameter D of the columnar body is 0.5 to 2.

5. A cellulose sponge according to claim 1, 2 or 3, characterized in that the cellulose sponge is of a columnar body for medical use and has the cut face almost vertical to the axial direction wherein the ratio L/D of the length L in the axial direction to the diameter D of the columnar body is 0.5 to 2.

6. A cellulose sponge according to claim 1, 2, 3 or 5, characterized in that the cellulose sponge is to be applied to medical use.

7. A method of producing a cellulose sponge according to claim 1, comprising:

preparing a raw sponge solution containing viscose and crystal mirabilite with no reinforcing fiber contained therein;
filling said raw sponge solution in a mold;
heating and solidifying for molding comprising a temperature holding process of holding said raw sponge solution at 35 °C or more to 70 °C or less for 30 minutes or more to 24 hours or less and subsequent complete solidification; and
post-treating to obtain the resulting product.

8. A method of producing a cellulose sponge according to claim 7, wherein the process for molding comprises a first temperature holding process of holding the raw sponge solution at 35°C or more to 70 °C or less for 30 minutes or more to 24 hours or less and a second temperature holding process at 90 °C or more to 100 °C or less after the first temperature holding process.

9. A method of producing a cellulose sponge according to claim 7 or 8, wherein the first temperature holding conditions carried out at the molding process are 35 °C or more to 45 °C or less for 30 minutes or more to 24 hours or less.

10. A method of producing a cellulose sponge according to claim 7, 8 or 9, wherein the molding process is carried out under no pressure applied.

11. A method of producing a cellulose sponge according to claim 7, 8, 9 or 10, wherein the average particle size of the crystal mirabilite is 0.01 mm or more to 0.2 mm or less.

12. A method of producing a cellulose sponge according to claim 7, 8, 9, 10 or 11, wherein polyalkylene glycol of an average molecular weight of 100 to 500 is contained in the raw sponge solution.

13. A method of producing a cellulose sponge according to claim 12, wherein the polyalkylene glycol is polyethylene glycol of an average molecular weight of 200 to 500.

14. A method of producing a cellulose sponge according to claim 7, 8, 9, 10, 11, 12 or 13, wherein at least one surfactant selected from sodium dodecyl sulfate, sorbitan monooleate ester and an ethylene oxide adduct of each of these surfactants is contained in the raw sponge solution.

15. A method of producing a cellulose sponge according to claim 7, 8, 9, 10, 11, 12, 13 or 14, wherein the mold to be used at the process for molding is a tubular mold.

16. A method of producing a cellulose sponge according to claim 7, 8, 9, 10, 11, 12, 13, 14 or 15, wherein the post-treatment process includes water washing process, cellulose regeneration process, desulfurization process, and bleaching process.

17. A method of producing a cellulose sponge according to claim 7, 8, 9, 10, 11, 12, 13, 14, 15 or 16, wherein the crystal mirabilite is added at an amount 50- to 100-fold to the weight of the cellulose in the viscose.

18. A method of producing a cellulose sponge according to claim 7, 8, 9, 10, 11, 12, 13, 14, 15 or 16, wherein the crystal mirabilite is added at an amount 60- to 80-fold to the weight of the cellulose in the viscose.

19. A method of producing a cellulose sponge according to claim 10, 11, 12, 13, 14, 15, 16, 17 or 18, wherein the polyalkylene glycol is polyethylene glycol and the amount thereof to be added is 2 to 4 % by weight to the cellulose weight in the viscose.

20. A method of producing a cellulose sponge according to claim 7, 8, 9, 10, 11, 12, 13, 14, 15, 16, 17, 18 or 19, wherein the viscose contains a type of cellulose of a polymerization degree of 200 to 400.

21. A method of producing a cellulose sponge according to claim 7, 8, 9, 10, 11, 12, 13, 14, 15, 16, 17, 18 or 19, wherein the viscose contains a type of cellulose of a polymerization degree of 250 to 350.

Fig. 1

F i g . 2

F i g . 3

F i g . 4

F i g . 5

Fig. 6

Fig. 7

## INTERNATIONAL SEARCH REPORT

| International application No. |
|---|
| PCT/JP97/01447 |

**A. CLASSIFICATION OF SUBJECT MATTER**

Int. Cl$^6$  C08J9/28, A61L31/00

According to International Patent Classification (IPC) or to both national classification and IPC

**B. FIELDS SEARCHED**

Minimum documentation searched (classification system followed by classification symbols)

Int. Cl$^6$  C08J9/00-9/28, A61L31/00, A61F13/00-13/20, A61L15/00

Documentation searched other than minimum documentation to the extent that such documents are included in the fields searched

| | |
|---|---|
| Jitsuyo Shinan Koho | 1926 - 1997 |
| Kokai Jitsuyo Shinan Koho | 1971 - 1997 |
| Toroku Jitsuyo Shinan Koho | 1994 - 1997 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 02-160843, A (Lion Corp.), June 20, 1990 (20. 06. 90), Claim; page 5, lower right column, line 4 to page 6, upper left column, line 19 (Family: none) | 1 - 21 |
| A | JP, 06-263911, A (Nisshinbo Industries, Inc.), September 20, 1994 (20. 09. 94), Claim; page 5, column 7, line 33 to column 8, line 16; Figs. 1 to 3 (Family: none) | 1 - 21 |
| A | JP, 02-135235, A (Lion Corp.), May 24, 1990 (24. 05. 90), Claim; page 1, lower right column, lines 9 to 16; page 2, lower right column, lines 5 to 11; page 3 to page 4, example 1 (Family: none) | 1 - 21 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| July 18, 1997 (18. 07. 97) | July 29, 1997 (29. 07. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)